# EUROPEAN PATENT APPLICATION

(11) **EP 4 455 115 A1**
(43) Date of publication of application: **30.10.2024**
(21) Application number: 22908943.8
(22) Date of filing: 16.12.2022
(51) Int. Cl.: C07C 6/04, C07C 67/03, C10G 45/00

(54) **INTEGRATED METHOD FOR PRODUCING LONG-CHAIN LINEAR OLEFINS AND AVIATION KEROSENE BY HOMOGENEOUS METATHESIS**

(30) Priority: 23.12.2021 BR 102021026313; 14.12.2022 BR 102022025516
(71) Applicant: Petroleo Brasileiro S.A. - PETROBRAS, 20031912 Rio de Janeiro (BR)
(72) Inventor: RABELLO, Carlos Rene Klotz, 21941-915 Rio de Janeiro, RJ (BR); JUNIOR, Marlito Gomes, 21941-915 Rio de Janeiro, RJ (BR)
(74) Representative: Clarke, Modet y Cía., S.L.
(86) International application number: PCT/BR2022/050506
(87) International publication number: WO 2023/115185

(57) **Abstract**

The present invention addresses to a process that comprises a step of selective hydrogenation of vegetable oil derivatives that are mixed with light olefins and sent to the metathesis section, where the renewable carbon content in the olefins and in the biojet fuel depends on the type and origin of the stream of light olefins. After the metathesis, the reactor effluent is sent to the separation and purification section, where there are obtained a stream of 1-decene (99% m/m), a stream of C11-C14 olefins (99% m/m) and a stream of C10-C14 methyl esters or carboxylic acids that is directed to co-processing together with jet fuel of fossil origin in an existing hydroprocessing unit. The light olefins are admitted in excess, and the unreacted part is separated and returned to the metathesis section. There is also a small formation of higher molecular weight compounds resulting from self-metathesis reactions that are separated and incinerated or used as fuel in furnaces or boilers to generate energy. The stream of saturated FAME (stearate and palmitate and methyl) present in the charge can be destined for co-processing (up to acceptable levels) or sold as biodiesel.

## Description

### Field of the Invention

The present invention addresses to a process aiming at the integrated production of long-chain linear olefins and aviation biokerosene from the metathesis of vegetable oils and their derivatives and streams of light olefins. More specifically, the renewable long-chain linear olefins generated by the present process can be used as raw materials for the production of detergents and lubricants, and the C10-C14 esters, after the hydrotreatment, can be used for the production of aviation biokerosene.

### Description of the State of the Art

The production of long-chain linear olefins is carried out, for the most part, through the Fischer-Tropsch processes and the ethylene oligomerization process (SHOP - Shell Higher Olefin Process). These are processes considered to have low selectivity and, therefore, requiring large capacity units, since the variety of streams and products obtained must be subsequently separated and purified. In this way, these are units that require large investments and high operating costs.

It should be emphasized that the raw materials used are of fossil origin, and renewable products are not obtained. In relation to the aviation biokerosene (biojet fuel), its production takes place in captive units, where more available vegetable oils, mainly soybean and palm oils, are hydroprocessed in a first step to obtain the respective paraffins. In a second step, due to the linearity and size of the chain, the paraffins formed must necessarily be subjected to an isomerization step, in order to meet the specification in terms of freezing point. Therefore, the investment in this unit is quite relevant, having a considerable impact on the final price of the product.

The global demand for products of renewable and sustainable origin has been growing as a way of reducing greenhouse gas emissions. On the other hand, faced with a future scenario of reduced gasoline consumption resulting from the electrification of the automobile fleet, oil companies have been allocating a significant part of their investments in the production of petrochemicals as a way of maintaining their profit margin. Furthermore, environmental legislation worldwide has been implemented with the aim of increasing the participation of renewable streams in petroleum derivatives. Thus, as happened with gasoline and diesel, the renewable content in aviation kerosene has been discussed. In Brazil, environmental agencies are studying the staggered addition of 3.4% vol/vol of aviation biokerosene (biojet fuel) to fossil kerosene by 2027.

Currently, there is no national production of biojet fuel. In this way, taking into account the previous premises, there is a need for a process aiming at the integrated production of long-chain linear olefins and biojet fuel from the metathesis of vegetable oils and their derivatives and streams of light olefins.

Document US20210171420A1 discloses a process for producing olefinic compounds and a hydrocarbon fuel or a fraction, which comprises subjecting a mixture of glycerides, having at least one unsaturated hydrocarbon chain, to a metathesis reaction and, after separation of the obtained olefinic mixture, carry out a hydrodeoxygenation process and subsequently hydroisomerization, in order to obtain the hydrocarbon fuel or its fraction. However, such a process jointly discloses the hydrotreatment and hydroisomerization of the stream of medium-chain esters.

Document US20170190983A1 discloses a process consisting of providing a renewable raw material comprising an unsaturated fatty acid glyceryl ester in contact with a mixture of the raw material and C2-C5 olefins with a metathesis catalyst, where the unsaturated fatty acid glyceryl ester and the C2-C5 olefin undergo a cross-metathesis reaction, thereby forming a metathesis product comprising medium-chain fatty acid esters, acyclic olefins, cyclic olefins and, optionally, C2-C5 olefins that did not react. However, such a process does not address to co-processing.

Document WO2021058876A1 refers to a process for the production of renewable products, such as renewable alkenes, for example, 1-decene, in particular to processes that include a metathesis reaction of an unsaturated fatty acid ester. Furthermore, there is provided a method related to the use of more efficient biomass in the production of alkenes with the desired carbon number through the use of the C=C double bonds that occur naturally in the raw material. However, such a process jointly discloses the hydrotreatment and hydroisomerization of the stream of medium-chain esters. Document MX2013008761A discloses a hydrocarbon fuel composition, including a derivative of a biological source selected from the group consisting of vegetable oil, animal fat and combinations thereof, and the hydrocarbons and the fuel composition are at least substantially free of oxygen, and a method for preparing a fuel composition. The method includes reacting a compound derived from a biological source selected from the group consisting of vegetable oils, animal fats and combinations thereof, with water to form free fatty acids; subjecting the free fatty acid to Kolbe electrolysis in the presence of an electrolyte, and removing an oxygen-containing carboxyl group from the free fatty acid to form a hydrocarbon. However, this method does not address to hydrogenation.

With this in mind, no document of the state of the art discloses a process that has high selectivity and yield in the olefins of greatest interest for the production of lubricants and detergents (C10-C14 chains) and, further, having as a co-product the biojet fuel capable of specifying the jet fuel in relation to its renewable content. Furthermore, this technology has great flexibility, as the size of the chain and the olefin production ratio will be directly linked to the type of vegetable oil and the size of the used light olefins.

In this way, with the aim of solving such problems, the present invention was developed through a process aiming at the integrated production of long-chain linear olefins and biojet fuel from the metathesis of vegetable oils and their derivatives and streams of light olefins. The olefins can be used as raw material for group IV lubricants (PAO - PolyAlphaOlefins) and detergents. The aviation kerosene already specified with the desired renewable content and freezing point is obtained from the co-processing of an intermediate stream of the metathesis process, together with kerosene of fossil origin in a conventional hydrotreatment unit for derivatives existing in refineries, thus minimizing investment in the construction of captive units for this purpose.

The invention can be used in the production of renewable long-chain linear olefins (raw material for the production of detergents and lubricants) and for the production of biojet fuel through the hydrotreatment of the formed C10-C14 esters.

An additional advantage is the possible co-processing of the formed stream of C10-C14 esters in existing hydroprocessing units in refineries, specifying the jet fuel with the desired content of renewable carbons and meeting future legislative demands (2027) and without removing the freezing point from the specification. The co-processing route allows for a significant reduction in investment by avoiding the construction of captive units for processing vegetable oils via the HEFA (Hydroprocessed Esters and Fatty Acids) route.

It is worth emphasizing that the co-processing of long-chain esters C16 and C18 (found in the most common vegetable oils such as soybean and palm) and the vegetable oils themselves must be less than 2% so as not to remove the freezing point of jet fuel from the specification. In this way, the predicted content of 3.4% vol/vol of biojet fuel in the final renewable jet fuel cannot be met by co-processing.

Thus, the present invention presents advantages that allow the production of jet fuel with renewable content in order to comply with future environmental legislation; valuation of the refinery C4 cut as products with high added value; availability of raw materials for the production of Group IV lubricants (PAO); sale of stream of long-chain linear olefins for the detergent segment; and production of streams of renewable origins.

### Brief Description of the Invention

The present invention addresses to an integrated process for obtaining a stream of long-chain linear olefins and a stream of C10-C14 esters that will be directed to co-processing in a hydrotreatment unit, generating aviation kerosene (jet fuel) with a renewable carbon content of around 3.5%, through the feeding of FAME (Fatty Acid Methyl Ester) and light olefins (C2-C5) of fossil origin.

In a second aspect, the invention aims at obtaining a stream of long-chain linear olefins and a stream of C10-C14 esters that will be directed to co-processing in a hydrotreatment unit, generating a jet fuel with a content of renewable carbon of around 3.6% through the joint feeding of light olefins (C2-C5) of fossil origin and ethylene of renewable origin obtained through the dehydration of ethanol and FAME aiming at maximizing the production of 1-decene and the increase of the renewable carbon content in the products.

In a third aspect, the invention aims at obtaining a stream of 1-decene and a stream of C10-C14 esters that will be directed to co-processing in a hydrotreatment unit, generating a jet fuel with a renewable carbon content of around 3.8% through feeding only, with ethylene from renewable sources obtained through the dehydration of ethanol in order to obtain products entirely from renewable sources.

### Brief Description of the Drawings

The present invention will be described in more detail below, with reference to the attached figures that, in a schematic way and not limiting the inventive scope, represent examples of embodiment thereof. In the drawings, there are:
Figure 1 illustrating a schematic of the integrated process for producing linear olefins and aviation biokerosene (partially renewable olefins);
Figure 2 illustrating a schematic of the integrated process for producing linear olefins and aviation biokerosene (maximization of renewable high-carbon 1-decene);
Figure 3 illustrating a scheme of the integrated process for producing linear olefins and aviation biokerosene (production of renewable 1-decene and biojet fuel);
Figure 4 illustrating the ruthenium (I)-based metathesis catalyst with the ligand R;
Figure 5 illustrating the ligand R referring to the metathesis catalyst in Figure 4, where R can be the chemical structure (II), which corresponds to the structure of the first generation Grubbs catalysts (GI), or the chemical structure (III), which corresponds to the structure of second generation Grubbs catalysts (GII);
Figure 6 illustrating another ruthenium (IV)-based olefin metathesis catalyst, where R1 and R2 are organic radicals. The radical R2 can be a hydrogen atom (H) or the organic radical NHCO₂ⁱBu (isobutyl formate);
Figure 7 illustrating the organic radical R1 of the metathesis catalyst shown in Figure 6. The radical R1 can assume two different chemical structures as shown: V and VI. When the radical R1 corresponds to structure V, the catalyst is known as a second-generation Hoveyda-Grubbs catalyst (HGII) and when the radical R1 corresponds to structures VI and VII, the catalysts are known as second-generation Hoveyda-Grubbs analog catalysts.

### Detailed Description of the Invention

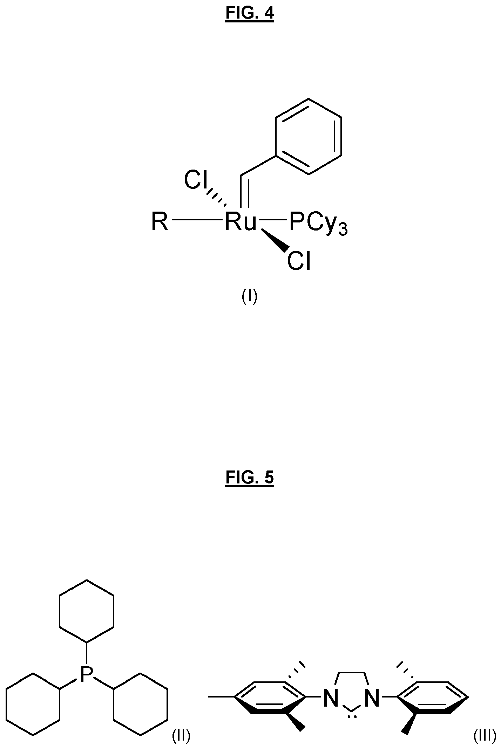

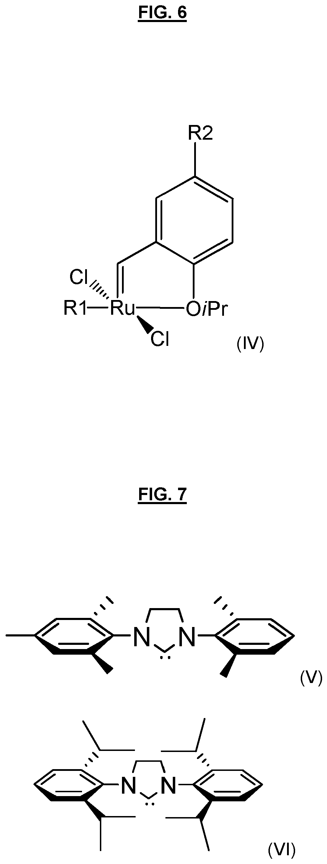

The process according to the present invention is based on the production of long-chain linear olefins and biojet fuel through the metathesis of vegetable oils and co-processing of formed esters, comprising the following steps:
Subjecting the stream of fatty methyl esters (1) obtained in the esterification of a vegetable oil to a step of Selective Hydrogenation of Esters (Charge) (A) using a catalyst from the family of palladium, palladium with silver and nickel supported on alumina;
Pre-treating the stream of light olefins (2) in the section of Contaminant Removal (B), wherein the contaminant removal can be carried out through aqueous washing, in liquid phase, in countercurrent, to remove nitrogenous compounds and cations and/or adsorption in a regenerative or non-regenerative fixed bed to remove oxygenated and sulfurated compounds;
After removing the contaminants, directing the treated stream of light olefins (3) to the section of Selective Hydrogenation of Light Olefins (C) for hydrogenation of dienes, if present, to their respective olefins, generating the stream (4); noble metal-based catalysts (e.g. palladium) or nickel supported on alumina are used for hydrogenation reactions; at the same time, drying the charge to increase process yield; and the hydrogenation can occur in the same reactor in which the fatty methyl esters are treated or in a segregated reactor;
After the respective treatments, the streams of fatty methyl esters of the charge and light olefins are mixed in appropriate proportions generating the feed stream (5) of the Homogeneous Metathesis process (D); if the stream of olefins (3) needs to be hydrogenated generating the stream (4), this stream can be fed directly to the Homogeneous Metathesis process (D); the Metathesis Process (D) uses a catalyst (9) that comprises a noble metal-based complex (as can be seen in Figures 4-7) solubilized in an organic solvent fed into a continuous flow stirred tank reactor (CSTR);
Sending the effluent from the metathesis reactor (6) to the section of Recovery of Light Olefins (E), where the stream of unreacted and recovered light olefins (10) is recycled to the metathesis reactor;
Sending the stream containing the products (7) formed in the metathesis to the section of Fractionation of Esters and Olefins (F) to be separated and purified, where there are obtained 1-Decene (11), a stream of C11-C14 linear olefins (12) and a stream containing the C10-C14 methyl esters (8);
Sending the stream containing the C10-C14 methyl esters (8) to be co-processed in the section of Co-processing of Esters (G) with aviation kerosene (jet fuel) of fossil origin (14), in a hydrotreatment unit, in the presence of hydrogen (13).

The streams of hydrogen used in steps (A), (C) and (G) are, preferably, of high purity (95-99.5% mol); however, streams with lower hydrogen contents can be used in the process.

Alternatively, the stream of C10-C14 esters can be processed pure in a hydrotreatment unit to be sold directly as biojet fuel with a high percentage of renewable carbon, without the need to be hydroisomerized.

The stream of saturated esters (21) is separated in the step of Fractionation of Esters and Olefins (F), wherein the stream (21) can be sold as biodiesel or sent to hydrogenate the fatty esters in a section of Selective Hydrogenation to their respective fatty alcohols and subsequently dehydrated in a section of Dehydration of Fatty Alcohols to linear olefins C16 to C18; this stream of olefins can be used as an organic base for the production of oil well drilling fluids.

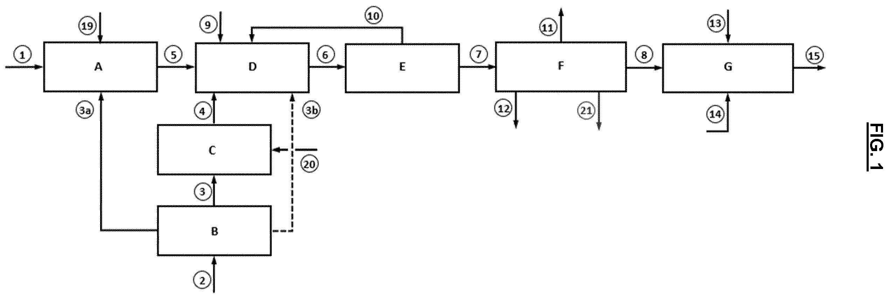

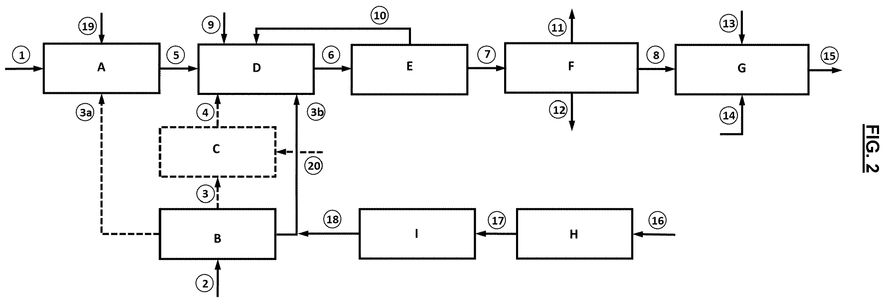

In a second embodiment of the invention (Figure 2), hydrated ethanol, preferably, and petrochemical grade 1-butene (99%) are fed to the integrated process with the aim of increasing the production of 1-decene; alternatively, together with 1-butene, there can also be fed a stream containing propylene (PROPINT - intermediate propylene stream coming from the FCC process); this configuration is very similar to the process shown in Figure 1, except for the source of light olefins and, therefore, only the differences between the two will be detailed; hydrated ethanol (16) is fed to a section of Dehydration (H) with the objective of transforming ethanol into ethylene through the loss of a water molecule, generating the stream (17); oxygenated by-products are generated concomitantly in this step; the dehydration occurs, preferably, in the vapor phase, using an alumina catalyst, in fixed bed reactors in series, at temperatures above 200 °C; the stream (17) is sent to the section of Separation and Purification of Ethylene (I) to remove oxygenated compounds (aldehydes, alcohols and ketones) and to be dehydrated, obtaining the stream (18) with an ethylene content greater than 99% and with a level of contaminants that does not cause loss of yield to the metathesis catalyst; the stream (2) composed of 1-butene and, alternatively, propylene is subjected to a drying process (B); this stream of moisture-free olefins is mixed with the stream of purified ethylene (18), generating the stream (3b) that feeds the step of Homogeneous Metathesis (D); as the FCC C4 cut was not used, there is no need for selective hydrogenation of the light olefins, minimizing the processing costs; in this way, the yield in the stream of 1-decene (11) is increased, in order to meet the desired production of the industrial plant; the streams of 1-decene (11) and C11-C14 olefins (12) will have a higher renewable carbon content compared to the scheme in Figure 1, and the jet fuel (15) obtained after the Co-processing of Esters (G) can reach a renewable carbon content of 3.6%, meeting the future Brazilian specifications for the addition of renewables and without removing the freezing point of this derivative from the specification. Alternatively, the stream of esters can be processed pure in a hydrotreatment unit to be sold directly as biojet fuel.

In a third embodiment of the invention (Figure 4), only hydrated ethanol is fed to the integrated process, preferably, with the aim of maximizing the production of 1-decene and obtaining products with a renewable carbon content of 100%, since light olefins are now of completely renewable origin; this configuration is very similar to the process shown in Figure 1, except for the source of light olefins and, therefore, only the differences between the two will be detailed; hydrated ethanol (16) is fed to a section of Dehydration (H) with the objective of transforming ethanol into ethylene through the loss of a water molecule, generating the stream (17); oxygenated by-products are generated concomitantly in this step; the dehydration occurs, preferably, in the vapor phase, using an alumina catalyst, in fixed bed reactors in series, at temperatures above 200 °C; the stream (17) is sent to the section of Separation and Purification of Ethylene (I) to remove oxygenated compounds (aldehydes, alcohols and ketones) and to be dehydrated, obtaining the stream (18) with an ethylene content greater than 99% and with a level of contaminants that does not cause loss of yield to the metathesis catalyst; this purified stream of ethylene (18) is mixed with the stream of olefins (3b) or even sent directly to the step of Homogeneous Metathesis (D); in this way, almost only 1-decene is produced by the stream (9) in order to meet the desired production of the industrial plant; C11-C14 olefins that may be formed, but in amounts that are not worth recovering, will be sent to be co-processed and transformed into biojet fuel; the stream (8) is basically composed of C10 methyl esters and is directed to the section of Co-processing; in this case, the stream (11) containing the 1-decene and the stream (8) containing the C10 ester will have a renewable carbon content of 100%, and the jet fuel (15) obtained after Co-processing of Esters (G) can reach a renewable carbon content of 3.8%, meeting the future Brazilian specifications for the addition of renewables and without removing the freezing point of this derivative from the specification. Alternatively, the stream of esters can be processed pure in a hydrotreatment unit to be sold directly as biojet fuel; alternatively, the stream of C10 esters can be processed pure in a hydrotreating unit to be sold directly as biojet fuel containing 100% renewable carbon.

The renewable carbon content in olefins and biojet fuel depends on the type and origin of the stream of light olefins. If ethylene is used from the ethanol dehydration process, the renewable carbon content will be 100%. For 1-butene and propylene (PROPINT) and olefins contained in the FCC C4 cut, this value lies in the range of 82 to 87% renewable, depending on the conversion and selectivity of the hydrogenation process of the FAME charge. The process comprises a step of selective hydrogenation of vegetable oil derivatives that are mixed with light olefins and sent to the metathesis section. In the case of the C4 (FCC) cut, the stream needs to be treated to remove heteroatom compounds and, subsequently, selectively hydrogenated, which may be in the same reactor as vegetable oil derivatives or in a captive reactor. After the metathesis, the reactor effluent is sent to the separation and purification section, where a stream of 1-decene (99% m/m), a stream of C11-C14 olefins (99% m/m) and a stream of C10-C14 methyl esters that is directed to co-processing together with the aviation kerosene of fossil origin in an existing hydroprocessing unit. Light olefins are admitted in excess, and the unreacted part is separated and returned to the metathesis section. There is also a small formation of higher molecular weight compounds resulting from self-metathesis reactions that are separated and incinerated or used as fuel in furnaces or boilers to generate energy. The stream of saturated FAME (stearate and palmitate and methyl) present in the charge can be destined for co-processing (up to acceptable levels) or sold as biodiesel.

In this way, the invention aims at specifying the jet fuel with a renewable percentage required in the future, in the case of Brazil in 2027, through co-processing (addition of 5% v/v of the stream of C10-C14 esters together with 95% v/v with fossil charge in existing unit) of the stream of medium-chain esters formed in the metathesis process. There is no need for hydroisomerization of the stream.

Accordingly, the investment in hydrotreatment (which would be the largest investment) becomes unnecessary, and a product with a lower production cost is obtained. The results (Table IV) of the freezing point analysis show that, practically, no variation occurs and, therefore, this property, considered critical, is within specification. As an example, the processing of more than 1% vol/vol of long-chain oils or esters already removes the jet fuel from the specification, requiring hydroisomerization of the resulting stream.

### EXAMPLES:

The following examples are presented in order to more fully illustrate the nature of the present invention and the way of practicing the same, without, however, being considered as limiting its content. Tables II to IV summarize the examples presented in this patent. Table II shows the operational conditions under which the example tests were carried out; Table III presents the mass balance of the main streams; and, finally, in Table IV the results of freezing point and renewable carbon content of the linear stream of olefins obtained.

### Example 1: Process 1 - Partially renewable olefins.

As can be seen in Figure 1, the objective of the process is to obtain a stream of long-chain linear olefins and a stream of C10-C14 esters that will be directed to co-processing in a hydrotreatment unit, generating a jet fuel with a renewable carbon content of around 3.5% through the feeding of light olefins (C2-C5) of fossil origin.

The stream of fatty methyl esters (1) obtained in the esterification of a vegetable oil was previously subjected to a step of Selective Hydrogenation of Esters (Charge) (A) with the aim of transforming di- and tri-unsaturated fatty chains into monounsaturated and, at the same time, the removal of present peroxides, considered contaminants for metathesis reactions.

A specific catalyst with a low palladium content (as can be seen in Figures 4-7) supported on alumina was used, in order to minimize the total saturation of the chains and the isomerization of the remaining double bond of the fatty methyl esters. The chain of esters with 18 carbon atoms with the double bond in position 9 was maximized in this step, as it is the one of greatest interest for the process. In this step, if necessary, the charge of fatty methyl esters may dry. In this example, methyl esters derived from soybean oil were used.

The stream of light olefins (2) can be composed of olefins with a chain size that can vary from 2 to 5 carbon atoms and must undergo pre-treatment in the section of Contaminant Removal (B) aiming at removing compounds containing heteroatoms (sulfur, oxygen and nitrogen) and cations (Na, K, Ca, etc.). As the most suitable olefins, there can be mentioned streams of high purity of ethylene and propylene (chemical or polymer grade), a stream of intermediate propylene (PROPINT - mixture of propylene and propane from catalytic or thermal cracking process), olefin C4 cut and C5 cut from refineries or petrochemical plants, among others.

The step of Contaminant Removal (B) will depend on the stream of light olefins used, and can be carried out using aqueous washing, in liquid phase, in countercurrent, to remove nitrogenous compounds and cations and/or adsorption in a regenerative or non-regenerative fixed bed to remove oxygenated and sulfurated compounds. Once the contaminants have been removed, the treated stream of light olefins (3) is directed to the section of Selective Hydrogenation of Light Olefins (C) to transform dienes, if present, into their respective olefins. This hydrogenation can be in the same reactor in which the fatty methyl esters are treated or in a segregated reactor. In this way, the stream of olefins (3a), resulting from the treatment in the step of Contaminant Removal (B), will be sent to the step of Selective Hydrogenation of Esters (Charge) (A). Dienes are contaminants for the metathesis catalyst, as it drastically reduces its activity. At the same time, the charge dries, also helping to increase the process yield. Hydrogen (19) and (20), preferably of high purity, is used in both steps (A) and (C) of selective hydrogenation. Depending on the butadiene content present in the stream of olefins (2), the Selective Hydrogenation of Olefins can become an uneconomical step, with the penalty of the catalyst activity being more interesting and, therefore, it can be sent directly to the metathesis section, after the step of Contaminant Removal (B).

Specifically, in this example, it was chosen to use as light olefins, a C4 cut of FCC (Fluid Catalytic Cracking), whose composition is found in Table I. The steps of contaminant removal considered were aqueous washing to remove acetonitrile and the non-regenerative adsorption to remove sulfurated compounds. The selective hydrogenation aimed at transforming 1,3-butadiene into butene and MAPD into propylene. In parallel, the isomerization reactions of 1-butene to 2-butene and of cis-2-butene to trans-2-butene occurred.

**Table I - Composition of the C4 cut (FCC).**

| **Component** | **%m/m** |
|---|---|
| Propane | 1.80 |
| Propylene | 3.03 |
| Isobutane | 31.31 |
| n-Butane | 15.13 |
| trans-2-Butene | 11.79 |
| 1-Butene | 12.23 |
| Isobutene | 15.10 |
| cis-2-Butene | 7.85 |
| 1,3-Butadiene | 0.43 |
| Isopentane | 0.52 |
| n-Pentane | 0.28 |
| C5+ | 0.42 |
| Sulfurates (mg/kg) | 86 |
| MAPD [#] (mg/kg) | 103 |
| Acetonitrile (mg/kg) | 56 |
| **Total** | **100** |

| | |
|---|---|
| [#] MAPD: methylacetylene, propadiene | |

After the respective treatments, the streams of fatty methyl esters of the charge and light olefins are mixed in appropriate proportions generating the feed stream (5) of the Homogeneous Metathesis process (D). The catalyst (described previously) (9) characterized by a noble metal-based complex solubilized in organic solvent is fed to the continuous flow stirred tank reactor (CSTR). In the process of metathesis, double bonds are broken and the formed radicals are recombined. The selected catalyst aims at maximizing the yield of long-chain linear olefins and fatty methyl esters in the C10-C14 range. The relationship between these compounds in the reactor effluent is directly linked to the type of light olefin used as charge. The effluent from the metathesis reactor (6) is sent to the section of Recovery of Light Olefins (E), since they are admitted in excess in relation to the fatty chains. In this way, the stream of unreacted and recovered light olefins (10) is recycled to the metathesis reactor in order to increase the process yield. In addition to the products of interest C10-C14, lighter products are generated resulting from the metathesis of components that were formed from the isomerization of the double bond of the fatty chain and heavier products formed by the self-metathesis of the fatty chains. These products are removed as secondary streams that were not shown in the process block diagram (Figure 1).

Then, the stream containing the products (7) formed in the metathesis is sent to the section of Fractionation of Esters and Olefins (F) to be separated and purified into streams of interest. Basically, there are obtained 1-Decene (11) (purity of 99.5% m/m), a stream of C11-C14 linear olefins (12) (purity of 99.5% m/m) and a stream containing C10-C14 methyl esters (8), which is sent to be co-processed in the section of Co-processing of Esters (G) with aviation kerosene (jet fuel) of fossil origin (14), in an existing hydrotreatment unit in a refinery, in the presence of hydrogen, preferably of high purity (13). Considering a content of 5% v/v of the stream of C10-C14 esters, in relation to the total feed charge and the average co-processing yield, a partially renewable final jet fuel (15) is obtained, containing a content of 3.5 % of renewable carbon, meeting the future Brazilian specifications for the addition of renewables and, at the same time, without removing the freezing point of this derivative from the specification (Table IV). Alternatively, the stream of C10-C14 esters can be processed pure in a hydrotreatment unit to be sold directly as biojet fuel with a high percentage of renewable carbon.

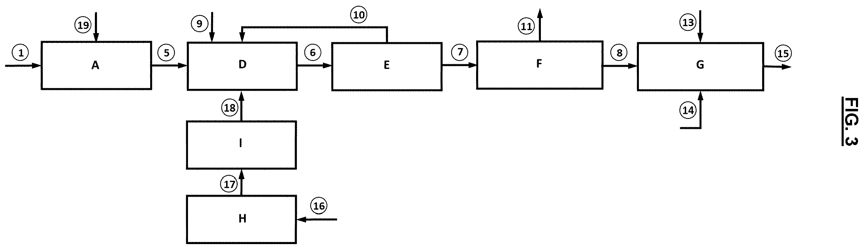

It is worth emphasizing that a stream of saturated esters (C16 and C18, in the case of palm and soybean oil) (21) is obtained, which are separated in the step of Fractionation of Esters and Olefins (F). This stream (21) can be sold as biodiesel or, more interestingly, this stream can be sent to hydrogenate the fatty esters to their respective fatty alcohols in a section of Selective Hydrogenation (not schematized) and subsequently dehydrated in a section of Dehydration of Fatty Alcohols (not schematized) to the respective linear olefins. In the case of producing C16-C18 olefins, their use would be as Drilling Fluid Organic Bases. Using esters from other oils that generate medium-chain olefins would be directed to detergents and lubricants (12), as can be seen in Figure 1. These observations about the unreacted stream of C16-C18 esters from charge (1) are also valid for Figures 2 and 3.

### Example 2: Process 2 - maximizing the production of 1-decene with a high renewable carbon content.

As can be seen in Figure 2, process 2 aims at obtaining a stream of long-chain linear olefins and a stream of C10-C14 esters that will be directed to co-processing in a hydrotreatment unit, generating a jet fuel with a renewable carbon content of around 3.6% through the joint feeding of light olefins (C2-C5) of fossil origin and ethylene of renewable origin obtained through the dehydration of ethanol aiming at maximizing the production of 1-decene and increasing the renewable carbon content in products.

The description of this process 2 is very similar to the process scheme 1 (Example 1), except for the source of light olefins. In this example, it was decided to feed hydrated ethanol (16) to a section of Dehydration (H) to the integrated process. Ethanol is dehydrated in fixed bed reactors in series, using alumina as a catalyst, obtaining an effluent (17) that is directed to the step of Distillation and Purification (I). In this step, there are basically removed oxygenated compounds formed during dehydration (aldehydes, ethers and ethanol) solubilized in decanted water, obtaining chemical grade ethylene. The chemical grade ethylene (99.0% m/m purity) was subjected to a subsequent cryogenic distillation step in order to obtain petrochemical grade ethylene (99.9% m/m purity). Ethylene (99.9% m/m purity) (18) from the ethanol dehydration process is characterized by being of completely renewable origin. Together with high-purity ethylene, it was decided to use petrochemical grade 1-butene (99.5% m/m purity) (2) as a second stream of light olefin of the integrated process. This stream of 1-butene was subjected to an adsorption step of Contaminant Removal (B) considered poisons for the metathesis catalyst. Since there was no need for pre-treatment of the streams of ethylene and 1-butene to be selectively hydrogenated to remove dienes, the resulting stream of light olefins (3b) was fed directly to the Homogeneous Metathesis process (D), together with the selectively hydrogenated stream of methyl esters (5). With this, there is obtained a greater production of 1-decene (11) in relation to the other generated streams of product. In this case, resulting from the feeding of these streams of light olefins, the stream (12) is basically composed of C12 olefins, whereas the stream of esters formed in metathesis (8) has only C10 and C12 esters. Further, the streams of 1-decene (11) and C10 and C12 methyl esters (8) will have a higher renewable carbon content compared to process 1 in Figure 1. The renewable jet fuel (15) obtained after Co-processing of Esters (G) can reach a renewable carbon content of 3.6%, meeting the future Brazilian specifications for the addition of renewables and, at the same time, without removing the freezing point of this derivative from the specification (Table IV). Alternatively, the stream of esters can be processed pure in a hydrotreatment unit to be sold directly as biojet fuel with a high renewable carbon content.

### Example 3: Process 3 - production of 100% renewable 1-decene and biojet fuel.

As can be seen in Figure 3, the objective is to obtain a stream of 1-decene and a stream of C10 esters that will be directed to co-processing in a hydrotreatment unit, generating a jet fuel with a renewable carbon content of around 3.8% through feeding only with ethylene from renewable sources obtained through the dehydration of ethanol aiming at obtaining products entirely from renewable sources.

The description of this process 3 is very similar to the process scheme 2 (Example 2), except for the source of light olefins. Only hydrated ethanol (16) is fed to the integrated process in the section of Dehydration (H), which consists of fixed bed reactors in series using alumina as a catalyst. An effluent containing ethylene resulting from the dehydration of ethanol (17) is obtained, which is sent to the step of Distillation and Purification (I). In this step, there are basically removed oxygenated compounds formed during dehydration (aldehydes, ethers and ethanol) solubilized in decanted water, obtaining chemical grade ethylene. The chemical grade ethylene (99.0% m/m purity) was subjected to a subsequent cryogenic distillation step in order to obtain petrochemical grade ethylene (99.9% m/m purity). High purity ethylene (18), characterized by being of totally renewable origin, is fed directly to the Homogeneous Metathesis process (D) with the selectively hydrogenated stream of methyl esters (5). With this, only 1-decene is obtained as a long-chain linear olefin and C10 ester as products, without the formation of considerable amounts of products with C11 and C12 carbon chains. The streams of 1-decene (11) and C10 methyl ester (8) will be 100% renewable, and the renewable jet fuel (15) obtained after Co-processing of Esters (G) can reach a renewable carbon content of 3.8%, meeting to the future Brazilian specifications for the addition of renewables and without removing the freezing point of this derivative from the specification (Table III). Olefins formed in the step of Homogeneous Metathesis (D) with more than 10 carbon atoms will be destined for co-processing, since the amount does not justify their purification. Alternatively, the stream of C10 esters can be processed pure in a hydrotreating unit to be sold directly as biojet fuel containing 100% renewable carbon.

The catalysts with the best performance were selected for the steps of selective hydrogenation of fatty methyl esters, light olefins and metathesis, as well as the optimization of process conditions. The stream of methyl esters (FAME) of soybean oil proved to be the most suitable raw material, but requires greater hydrogen consumption and greater care in the step of selective hydrogenation.

In terms of light olefins, ethylene was explored, which gives greater yield to 1-decene, 1-butene, which basically produces 1-decene and 3-dodecene, propylene present in the stream of PROPINT, which maximizes the production of 1-decene and 2-undecene and a C4 cut from the refinery FCC (Fluid Catalytic Cracking) process whose distribution of C10 to C14 olefins varies according to their composition.

Although the C4 cut has a price well below that of ethylene and 1-butene, there is greater expense in its purification to remove dienes and compounds containing heteroatoms. The stream of PROPINT has a similar cost to the FCC C4 cut, being an alternative that increases the profitability of the process. The ethylene used in the examples was of renewable origin, coming from the dehydration of ethanol; however, the ethylene of fossil origin will show similar behavior in the step of metathesis.

**Table II - Operating conditions of the proposed Process Schemes.**

| **Operational Conditions** | **Selective Hydrogenation of Esters (charge)** | **Selective Hydrogenation of Dienes of Light Olefins** | **Homogeneous Metathesis** | **Co-processing of Esters (product)** |
|---|---|---|---|---|
| Temperature (°C) | 80 | 35 | 60 | 325 |
| Pressure (bar (× 100 kPa)) | 5 | 12 | 7 | 52 |
| V.E.(h⁻¹) | 5 | 5 | 0.75 | 3.95 |
| H₂/charge ratio | 1.0 mol_{H2}/UNSAT [#] | 1.5 mol_{H2}/mol_{DIENES} | - | 100 vol_{H2}/vol_{CHARGE} |
| CAT/charge ratio | - | - | TON = 92.955 s⁻¹ | - |
| OL/charge ratio [§] | - | - | 2.5 mol_{OL}/mol_{ESTERS} | - |
| Catalyst | 0.1%PdAg/Al₂O₃ | 0.7%Pd/Al₂O₃ | Hoveyda-Grubbs II | NiMo |

| | | | | |
|---|---|---|---|---|
| [ § ] OL - Light olefins (C2-C5); [ # ] UNSAT - unsaturation, double bond present in the component chain. | | | | |

**Table III - Mass balance of the proposed Process Schemes.**

| **Denomination** | **Example (1)** | | **Example (2)** | | **Example (3)** | |
|---|---|---|---|---|---|---|
| | **Stream** | **kg/h** | **Stream** | **kg/h** | **Stream** | **kg/h** |
| FAME | 1 | 132.011 | 1 | 133.552 | 1 | 134.639 |
| Light Olefins | 4 | 19.194 | 3b | 13.507 | 3b | 11.290 |
| Esters | 8 | 66.549 | 8 | 65.722 | 8 | 65.444 |
| 1-Decene | 11 | 28.930 | 11 | 31.380 | 11 | 45.655 |
| Olefins C11-C12 | 12 | 20.690 | 12 | 16.861 | 12 | 0.000 |
| Renewable jet fuel(m³/h) | 15 | 1,402 | 15 | 1,385 | 15 | 1,378 |

**Table IV - Results of the renewable freezing point and carbon content of the stream of renewable jet fuel.**

| **Co-processing with jet fuel of 5% vol/vol of C10-C12 esters** | | |
|---|---|---|
| **Example** | **Freezing Point** | **% Renewable Carbon** |
| Pure jet fuel | -56.7 °C | - |
| 1 | -55.7 °C | 3.5 |
| 2 | -55.5 °C | 3.6 |
| 3 | -56.0 °C | 3.8 |

It should be noted that, although the present invention has been described in relation to the attached drawings, it may undergo modifications and adaptations by technicians skilled on the subject, depending on the specific situation, but as long as they are within the inventive scope defined herein.

## Claims

1. **AN INTERGRATED PROCESS FOR PRODUCING LONG-CHAIN LINEAR OLEFINS AND AVIATION BIOKEROSENE FROM HOMOSENEOUS METATHESIS, characterized in that** it comprises the following steps:
(a) Subjecting the stream of fatty methyl esters (1) obtained from the esterification of a vegetable oil to a step of Selective Hydrogenation of Esters (Charge) (A) using a specific catalyst with a low palladium content supported on alumina;
(b) Pre-treating the stream of light olefins (2) in the section of Contaminant Removal (B);
(c) After removing the contaminants, directing the treated stream of light olefins (3) to the section of Selective Hydrogenation of Light Olefins (C) to transform dienes, if present, into their respective olefins; at the same time, drying the charge to increase the process yield;
(d) After the respective treatments, the streams of fatty methyl esters of the charge and light olefins are mixed in appropriate proportions generating the feed stream (5) of the Homogeneous Metathesis process (D), using a catalyst (9) comprising a noble metal-based complex solubilized in organic solvent fed to the reactor;
(e) Sending the effluent from the metathesis reactor (6) to the section of Recovery of Light Olefins (E), where the stream of unreacted and recovered light olefins (10) is recycled to the metathesis reactor;
(f) Sending the stream containing the products (7) formed in the metathesis to the section of Fractionation of Esters and Olefins (F) to be separated and purified, where there are obtained 1-decene (11), a stream of C11-C14 linear olefins (12) and a stream containing the C10-C14 methyl esters (8);
(g) Sending the stream containing the C10-C14 methyl esters (8) to be co-processed in the section of Co-processing of Esters (G) with aviation kerosene (jet fuel) of fossil origin (14), in a hydrotreatment unit, in the presence of hydrogen (13).

2. **THE PROCESS** according to claim 1, **characterized in that** the step of Contaminant Removal (B) is carried out using aqueous washing, in liquid phase, in countercurrent, to remove nitrogenous compounds and cations and/or adsorption in a regenerative or non-regenerative fixed bed for removing oxygenated and sulfurated compounds.

3. **THE PROCESS** according to claim 1, **characterized in that** the hydrogenation of the section of Selective Hydrogenation of Light Olefins (C) can occur in the same reactor in which the fatty methyl esters are treated or in a segregated reactor.

4. **THE PROCESS** according to claim 1, **characterized in that** the hydrogen used in steps (A), (C) and (G) is, preferably, of high purity (90-99.9% mol).

5. **THE PROCESS** according to claim 1, **characterized in that** the reactor of the section of Homogeneous Metathesis (D) is a continuous flow stirred tank reactor (CSTR).

6. **THE PROCESS** according to claim 1, **characterized in that**, alternatively, the stream of C10-C14 esters is processed pure in a hydrotreatment unit to be sold directly as biojet fuel with a high percentage of renewable carbon, between 80 and 100%.

7. **THE PROCESS** according to claim 1, **characterized in that** a stream of saturated esters (21) is separated in the step of Fractionation of Esters and Olefins (F), wherein the stream (21) can be sold as biodiesel, or the stream can be sent to hydrogenate the fatty esters in the section of Selective Hydrogenation (J) to their respective fatty alcohols and, subsequently, dehydrated in the section of Dehydration of Fatty Alcohols (H) to olefins.

8. **THE PROCESS** according to claims 1 to 7, **characterized in that** there is, alternatively, fed to the integrated process hydrated ethanol (16) to a section of Dehydration (H), wherein the ethanol is dehydrated in fixed bed reactors in series, using alumina as a catalyst, obtaining an effluent (17) that is directed to the step of Distillation and Purification (I), for removal of present oxygenated compounds, dehydration and purification, obtaining an ethylene content greater than 95%.

9. **THE PROCESS** according to claim 8, **characterized in that** the C4 cut is selectively hydrogenated to remove dienes; the resulting stream of light olefins (3b) is fed directly to the Homogeneous Metathesis process (D), together with the selectively hydrogenated stream of methyl esters (5).

10. **THE PROCESS** according to claims 1 to 7, **characterized in that** there is, alternatively, fed to the integrated process only hydrated ethanol (16) to the section of Dehydration (H), which consists of fixed bed reactors in series using alumina as catalyst, obtaining an effluent containing ethylene resulting from the dehydration of ethanol (17) which is directed to the step of Distillation and Purification (I); where high purity ethylene (18) is fed directly to the Homogeneous Metathesis process (D) with the selectively hydrogenated stream of methyl esters (5), obtaining only the streams of 1-decene (11) and C10 methyl ester (11) and renewable jet fuel (15) obtained after the Co-processing of Esters (G) with renewable carbon content.
